# EUROPEAN PATENT SPECIFICATION

(11) **EP 1 371 773 B1**
(45) Date of publication and mention of the grant of the patent: **07.06.2006**
(21) Application number: 02012985.4
(22) Date of filing: 12.06.2002
(51) Int. Cl.: D06M 13/02, D06M 13/224, D06M 13/144, D06M 15/347, D06M 15/53, D06M 15/227, D06M 15/643, A61L 15/20, A61L 15/34

(54) **Lotions for nonwovens**
Lotionen für Vliesstoffe
Lotions pour non-tissés

(43) Date of publication of application: 17.12.2003
(73) Proprietor: Cognis Deutschland GmbH & Co. KG, 40789 Monheim (DE)
(72) Inventor: Wild, Christine, Dr., 40724 Hilden (DE); Mathis, Raymond, Dr., 40627 Düsseldorf (DE); Neuss, Michael, Dr., 50997 Köln (DE)

(56) References cited:
- WO-A-01/22933
- WO-A-97/05909
- GB-A- 1 564 363
- US-A- 5 106 656
- US-A- 5 723 137

## Description

The present invention relates to compositions for the finishing of nonwovens, and the use of selected compounds as crystallisation accelerators in such lotions, and to nonwovens which have been finished with the lotions according to the invention.

In the preparation of hygiene articles, such as nappies or sanitary towels, absorbing materials are used in order to absorb aqueous fluids. In order to prevent direct contact with the absorbing material upon wearing and to increase the wear comfort, this material is covered with a thin, water-pervious nonwoven. Nonwovens of this type are usually prepared from synthetic fibres, such as polyolefin or polyester fibres, since these fibres can be produced at low cost, have good mechanical properties and are thermally stable.

In the hygiene article sector, nonwovens of this type are increasingly provided with skin-friendly lotions in order to generally improve tolerability and wear comfort. For example, DE 33 09 530 C1 describes an hygienic absorption liner which is impregnated with a skincare material which consists of triglycerides and/or partial glycerides of coconut oil fatty acids having 8 to 18 carbon atoms. In order that these preparations can also transfer from the nonwoven to the skin without problems while being worn, the triglyceride and partial glyceride mixtures of DE 33 09 530 are chosen such that they have a rise point in the range from 35 to 40°C.

Another approach to transferring skincare substances to the skin during the wearing of hygiene articles is given in WO 96/16682. This describes a nappy whose inner covering web is prepared with a lotion which is solid or semisolid at 20°C and which transfers to the skin of the wearer while being worn. These lotions comprise from 10 to 95% of an anhydrous emollient which has to be plastic or liquid at room temperature, and 5 to 90% of a so-called immobilizer which is to have a melting point of at least 35°C, but preferably 40°C.

However, the main problem of the known lotions is their storage stability. It is essential that the lotions themselves are in a form at room temperature, i.e. approximately 36 to 38°C, such that they can transfer to the skin from the nonwoven without difficulties, i.e. at these temperatures the lotion should be sufficiently viscous to be detached from the nonwoven and transferred onto the skin. This temperature-dependent process can, however, lead to problems if the hygiene products are stored at relatively high temperatures, for example more than 30°C. In this case, it is frequently observed that the lotions "exude" on the nonwovens. It was therefore the object of the present invention to provide skin-friendly lotions for application to nonwovens for hygiene articles, where the storage stability of said lotions has to be ensured, in particular at high temperatures.

Furthermore, it is to be noted that the nonwoven in, for example, nappies must be impervious to liquids and has therefore usually been prepared to be hydrophilic. The further finishing with a usually hydrophobic skin-friendly lotion could therefore reduce or significantly impair the transportation of liquid through the web into the absorbing materials.
Furthermore, it is desired that the lotions transfer as completely as possible from the nonwoven onto the skin of the wearer and, in this connection, optionally provide further additional uses, for example are able to reduce the formation of an odour, or else the growth of bacteria, fungi and yeasts. In principle, it must of course be possible to apply the lotions to the nonwovens easily and to apply them as far as possible using the known preparation processes. It has been found that these properties could not be achieved in totality using the products of the prior art.

Surprisingly, it has been found that by combining three components chosen on the basis of their melting behaviour, it is possible to achieve the above object.

The present invention thus provides, in a first embodiment, a composition solid at 21°C comprising at least
a) 5 to 70% by weight of a component melting in the range from 25 to 37°C, chosen from the group of synthetic waxes, paraffins, fatty acid esters, polyhydroxy fatty acid esters, fatty alcohols, alkoxylated fatty acid esters, alkoxylated fatty alcohols and mixtures of these compounds and
b) 5 to 70% by weight of a component whose melting point is at least 5°C higher than the melting point of component a), and component b) is chosen from the group of paraffins, polyhydroxy fatty acid esters, C14-22 fatty alcohols, C12-22 fatty acids, the alkoxylated derivatives of the fatty alcohols and fatty esters, and mixtures of these components, where the compositions additionally comprise as component
c) 5 to 25% by weight of a crystallisation accelerator chosen from the group of partial glycerides, ethylene glycol diesters and polymeric waxes, with the proviso that the crystallisation accelerator has a melting point in the range from 45 to 70°C, and that the compositions comprise less than 5% by weight of water.

The compositions according to the invention obligatorily comprise three constituents, being characterised, in particular, by the presence of component c), a crystallisation accelerator. Furthermore, it is essential to chose components a) to c) according to their melting behaviour.

Melting itself is defined as the transition of a substance from the solid to the liquid aggregate state by the input of thermal energy, where, as a consequence of an increase in kinetic energy of the particles, their oscillation amplitude becomes so great that the lattice structure collapses. The melting point is defined as the temperature at which the liquid and the solid phase of a substance are in thermodynamic equilibrium at a pressure of, normally, 1013 hPa. In actual fact, the term "melting point" is, however, used in practice mostly only for the transition point from the solid state to the liquid state, and not for the temperature, identical thereto, at which the transition in the reverse direction takes place. The amount of heat absorbed in this process is referred to as the heat of melting or the enthalpy of melting. Usually, the melting point increases with increasing pressure, although there are exceptions. For many pure substances, the melting points can be determined with great accuracy since here the temperature remains constant over a certain time interval during the introduction of heat. For amorphous, glass-like substances, there is no specific melting point since there are no crystal lattices here. Similar phenomena are observed in the melting behaviour of fats, ointments, creams or suppository materials; in such cases, it is possible to use the solidification point, the so-called rise point and the dropping point for characterisations.

The action of this crystallisation accelerator is to be understood as meaning it has a very sharp melting point which must be higher than the melting point of the liquid to semiliquid component a), but should be lower than the temperature at which the lotion is applied. The combination of component a), which melts at relatively low temperatures, with component b), which melts at higher temperatures, and the simultaneous addition of the crystallisation accelerator c) gives lotions which, firstly, are still storage-stable even at high temperatures, and at the same time can be transferred from the nonwoven to the skin of the wearer while being worn.

Component a) can be chosen from a large number of compounds known to the person skilled in the art, it being essential that the melting point here must be in the range from 25 to at most 37°C. Firstly, for this purpose it is possible to use certain paraffins, but also fatty acid esters and, in particular, fatty alcohols. Suitable paraffins are preferably semisolid paraffins, such as soft paraffin, preferably petrolatum. Suitable fatty alcohols are, for example, dodecanol or ricinol alcohol, to name one representative of the unsaturated fatty alcohols. Further suitable substances are chosen from the class of synthetic waxes, for example copolymers of polyethylene/maleic anhydride.
For the purposes of the present invention, the use of glycerides is particularly suitable, here preferably the mixtures of partial glyceride and triglycerides, which must have the desired melting point of from 25 to 37°C. Particular preference is given here to mixtures of glycerides of fatty acids having 8 to 18 carbon atoms.

Glycerides are mono-, di- and/or triesters of glycerol with fatty acids, namely, for example, caproic acid, caprylic acid, 2 ethylhexanoic acid, capric acid, lauric acid, isotridecanoic acid, myristic acid, palmitic acid, palmoleic acid, stearic acid, isostearic acid, oleic acid, elaidic acid, petroselic acid, linoleic acid, linolenic acid, elaeostearic acid, arachidic acid, gadoleic acid, behenic acid and erucic acid, and technical-grade mixtures thereof.

They conform to the formula (I), 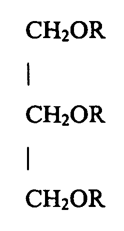 in which R is a COR' radical, in which R' is a branched or unbranched, saturated or unsaturated alkyl radical having 6 to 22 carbon atoms, and/or independently thereof, is hydrogen. Typical examples are lauric acid monoglyceride, lauric acid diglyceride, coconut fatty acid monoglyceride, coconut fatty acid triglyceride, palmitic acid monoglyceride, palmitic acid triglyceride, stearic acid monoglyceride, stearic acid diglyceride, isostearic acid monoglyceride, isostearic acid diglyceride, oleic acid monoglyceride, oleic acid diglyceride, tallow fatty acid monoglyceride, tallow fatty acid diglyceride, behenic acid monoglyceride, behenic acid diglyceride, erucic acid monoglyceride, erucic acid diglyceride, and technical-grade mixtures thereof, which may also comprise small amounts of triglyceride as secondary components from the preparation process.

Suitable as component a) are, in particular, those mixtures of coconut partial glyceride and triglycerides sold under the trade name Novata(B by the applicant. Novata B has a melting point (in accordance with DGF CIV 3a) of from 33 to 36°C, and the acid number is 0.3 (in accordance with DGF CV2). The saponification value is 225-240 in accordance with DGFCV3. The molecular weight is 710. A further suitable mixture for component a) is Novata(299 (melting point 34°C) from the applicant.

Component b) must have a melting point which is at least 5°C higher than the melting point of component a) used in the composition in question. Preferably component b) has a melting point in the range from 40 to 60°C.
Particular preference is given here to those compounds of the group of C 14-22-fatty alcohols, C12-22-fatty acids and alkoxylated derivatives thereof, and fatty alcohols and esters. However, hard paraffins having the desired melt properties are also suitable. In this case too, the mixtures of different glycerides or fatty acids of the C8-18 cut, in particular, are suitable as component b) for the purposes of the present technical teaching. Very particularly suitable both for component a) and also b) are glycerol triesters and partial esters of coconut fatty acids. These are the C8-C18 cut, where, depending on the choice of chain lengths of the glycerides or of the degree of esterification, it is possible to create mixtures with varying melting points. Mixtures of lauric (C14) and myristic (C16) esters are essentially present.

The higher-melting component b) used is preferably Novata D from the applicant. This too is a mixture of triglycerides and partial glycerides of coconut fatty acids, but with a different melting range. Novata D has a melting point (in accordance with DGF C IV 3a) of from 40 to 42°C, and the acid number is 0.3 (in accordance with DGF C V 2). The saponification value is 215-230 (in accordance with DGF C V 3).

The use of the crystallisation accelerator component c) is essential for the present invention. This is a substance with a melting range which is, firstly, at least 45°C, and, secondly, the melting range of this component should, however, preferably be as narrow as possible and should thus not extend over more than 4.5°C, preferably 2.5°C, and below.

Particularly suitable as component c) here are selected glycerol partial esters with C12-C21-fatty acids, preferably glycerol monostearate, having a melting range in accordance with DGF C IV 3a of 58-60°C, or glycerol monolaurate, melting range according to DGF IV 3a of 56-60°C. Further suitable monoglycerides are glycerol monocaprate (melting point 53°C) or glycerol myristate (melting point 70.5°C).

If the crystallisation accelerator c) according to the invention is used, lotions are obtained which can be applied to the nonwovens without problems since their melting temperature is higher than that of the lower-melting component a), but is not so high that it reaches the application temperature of the lotion onto the nonwovens. The application temperature is preferably 60-80°C, it being necessary to also adapt the application temperature, depending on the crystallisation accelerator used.

In selecting components a) to c), their melting points are, firstly of significance. In addition, it is to be taken into consideration that at least three different substances are present in the composition according to the invention according to the definition of component a) to c). Component c) preferably has a melting point in the range from 30 to 35°C. It is particularly preferred if component c) has a melting point below the melting point of component b) and is preferably 5 to 10°C, in particular 10 to 15°C, lower.

The compositions themselves preferably have a melting point in the range from 35 to 65°C, in particular from 35 to 50°C and preferably 35 to 45°C. The melting behaviour of the compositions is essential for the use, and it is therefore preferred for the compositions according to the invention to have two melting ranges which are clearly separate from one another, as can preferably be ascertained by means of DSC measurements. The enthalpies of melting for the compositions are preferably 80 to 160 J/g, in particular 90 to 140 J/g and particularly preferably 100 to 125 J/g. Furthermore, the co-use of liquid or semisolid compounds, as disclosed in the above-cited WO 96/16682, and also in WO 96/16681, is excluded.

The compositions according to the invention are anhydrous, i.e. they comprise water in a total amount of less than 5% by weight, preferably 0.5 to 3 % by weight, more preferably 0.5 to 2 % by weight and in particular 0.5 to 1.5% by weight. Accordingly, anhydrous components a) to c) are preferably to be chosen in order to avoid expensive drying steps during the preparation.

In the compositions according to the invention, in a preferred embodiment, component a) is present in amounts of from 10 to 60% by weight, component b) is present in amounts of from 10 to 60% by weight and component c) is present in amounts of from 10 to 25 % by weight.

In addition, further customary ingredients may also be present in the compositions according to the invention, for example silicone waxes or polysiloxanes, in amounts of from 1 to 6% by weight, preferably 1.5 to 5.5% by weight and in particular from 2 to 5% by weight. Polysiloxanes are known polymeric compounds which contain the following structure as monomer units:

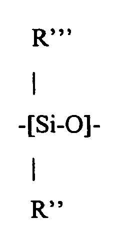

Here, R' and R"', independently of one another, are hydrogen or an alkyl, cycloalkyl, aryl or alkenyl radical. Siloxanes of this type preferably have viscosities at 37°C in the range from 5 to 5 000 mPa s.

In addition, the compositions according to the invention may comprise skin-friendly or skincare substances, preferably in amounts of from 0.1 to 10% by weight, in particular 1 to 8% by weight and very particularly preferably from 2 to 6% by weight.

Ingredients of this type may, for example, be bisabolol, alantoin and panthenol. It is also possible to use vitamins, preferably vitamin E and vitamin precursors, and protein hydrolysates. Also suitable are plant extracts, preferably from camomile, aloe vera, lime blossom, horse chestnut, green tea, oak bark, stinging nettle, hops, burdock, horsetail, hawthorn, almond, spruce needle, almond wood, juniper, coconut, apricot, lemon, wheat, kiwi, melon, orange, grapefruit, sage, rosemary, birch, mallow, lady's smock, thyme, balm, restharrow, coltsfoot, althea, ginseng and root ginger. In addition, however, other skincare substances may also be present. Those which may be named here are, in particular, chitosan, and zinc oxide or zinc ricinoleate.

A particularly preferred composition of the present invention comprises 50 to 60% by weight of a mixture of glycerol esters of coconut fatty acids having a melting point of from 30 to 33°C as component a), 10 to 20% by weight of a linear, unsaturated fatty alcohol having a melting point of from 57 to 60°C as component b), 15 to 20 parts of polyvinyl stearyl ether having a melting point of from 45 to 48°C as component c), and optionally 2 to 5 % by weight of silicone wax, and optionally 5 to 10% by weight of a skincare substance, preferably of avocado oil.

The compositions are prepared in a manner customary per se, by mixing the individual liquid components, i.e. at elevated temperatures, preferably at 40 to 80°C and in particular at 50 to 70°C. A particular sequence during the mixing of the components is not necessary. The compositions are then cooled to room temperature (21°C).

The present invention further provides for the use of glycerol monolaurate and/or polyvinyl stearyl ether as crystallisation accelerator in lotions for the skin-friendly finishing of nonwovens.

The nonwovens are substances known to the person skilled in the art. For the purposes of the present invention, preferred nonwovens are those which consist entirely or partially of polyolefins. Suitable for this are all types of polymer and copolymer known to date and based on ethylene or propylene. Mixtures of pure polyolefins with copolymers are also suitable in principle. Types of polymer which are particularly suitable for the teaching according to the invention are listed below: poly(ethylenes), such as HDPE (high density polyethylene), LDPE (low density polyethylene), VLDPE (very low density polyethylene), LLDPE (linear low density polyethylene), MDPE (medium density polyethylene), UHMPE (ultra high molecular polyethylene), CPE (crosslinked polyethylene), HPPE (high pressure polyethylene); isotactic polypropylene; syndiotactic polypropylene; polypropylene prepared under metallocene catalysis, impact-modified polypropylene, random copolymers based on ethylene and polypropylene, block copolymers based on ethylene and polypropylene; EPM (poly[ethylenepropylene]); EPDM (poly[ethylenepropylene conjugated diene]). Further suitable types of polymer are: poly(styrene); poly(methylstyrene); poly(oxymethylene); metallocene-catalysed alpha-olefin or cycloolefin copolymers, such as norborneneethylene copolymers; copolymers which contain at least 80% of ethylene and/or styrene and less than 20% of monomers such as vinyl acetate, acrylic ester, methacrylic ester, acrylic acid, acrylonitrile, vinyl chloride. Examples of such polymers are: poly(ethyleneethyl acrylate), poly(ethylenevinyl acetate), poly(ethylenevinyl chloride), poly(styreneacrylonitrile). Also suitable are graft copolymers and polymer blends, i.e. mixtures of polymers in which, inter alia, the abovementioned polymers are present, for example polymer blends based on polyethylene and polypropylene.

Within the scope of the present invention, homopolymers and copolymers based on ethylene and polypropylene are particularly preferred. In one embodiment of the present invention, polyethylene is accordingly used exclusively as polyolefin, in another embodiment polypropylene is used exclusively, and in another embodiment copolymers based on ethylene and polypropylene are used.

In a very particularly preferred embodiment of the invention, component a) is polypropylene. The invention further provides for the use of hydrophilicized polyolefin- or polyester-based fibres prepared by the above-described process and wettable by aqueous media for producing textile fabrics. Preferably, the textile fabrics are nonwovens. In a particularly preferred embodiment, these textile fabrics are intended for use in nappies.

The present invention also includes the technical teaching of finishing nonwovens which contain polyethylenes with compositions according to the above description.

### Examples

The following compositions 1 and 2 according to the invention were prepared and their melting behaviour was investigated. For this purpose, DSC measurements were carried out in each case. The heating/Cooling rates were 10K/min and -1 K/min respectively.

### Composition 1:

a) 58% by weight of a mixture of partial glycerides and triglycerides of coconut fatty acids, melting point: 34°C
b) 15% by weight of stearyl alcohol, melting point 56-58°C
c) 20% by weight of polyvinyl stearyl ether, melting point 45°C
d) 2% by weight of silicone wax

The melting point of the composition was 49°C. The heat of melting was 112 J/g.

### Composition 2:

a) 55% by weight of a mixture of partial glycerides and triglycerides of coconut fatty acids, melting point: 34°C
b) 15% by weight of glycerol monolaurate, melting point 63°C
c) 20% by weight of polyvinyl stearyl ether, melting point 45°C
d) 5% by weight of silicone wax

The melting point was 46°C. The heat of melting was 82 J/g.

The compositions described above can be applied to nonwovens without problems and are suitable for the preparation of nonwovens for hygiene products.

To determine the cooling characteristic of lotions according to the present invention three lotions were prepared an heated to 70 °C. Lotions 1 and 2 contain crystallisation accelerators according to the invention. Lotion 3 is free of this compound.

The hot lotions were put (0.5 ml each) on a slanting glass plate (angle 35°). Then the distance was measured, until the drop comes to stop. For lotion 1 and 2 a distance of approximately 15 cm was measured, the lotion without compound c) according to claim 1 needs 21 cm to come to stop.

## Claims

1. Composition solid at 21°C comprising at least
a) 5 to 70% by weight of a component melting in the range from 25 to 37°C, chosen from the group of synthetic waxes, paraffins, fatty acid esters, polyhydroxy fatty acid esters, fatty alcohols, alkoxylated fatty acid esters, alkoxylated fatty alcohols and mixtures of these compounds and
b) 5 to 70% by weight of a component whose melting point is at least 5°C higher than the melting point of component a), and component b) is chosen from the group of polyhydroxy fatty acid esters, C14-22 fatty alcohols, C12-22 fatty acids, the alkoxylated derivatives of the fatty alcohols and fatty esters, and mixtures of these components, **characterised in that** the compositions additionally comprise as component
c) 5 to 25% by weight of a crystallisation accelerator chosen from the group of partial glycerides and polymeric waxes, with the proviso that the crystallisation accelerator has a melting point in the range from 45 to 70°C, and that the compositions comprise less than 5% by weight of water.

2. Composition according to Claim 1, **characterised in that** component b) has a melting point in the range from 40 to 60°C.

3. Composition according and Claims 1 and 2, **characterised in that** component a) is chosen from the mixtures of glycerides of fatty acids having 8 to 18 carbon atoms, preferably from technical-grade mixtures of partial glycerides and/or mixtures with glycerides.

4. Composition according to Claims 1 to 3, **characterised in that** component b) is chosen from mixtures of glycerides of fatty acids having 8 to 18 carbon atoms, preferably from technical-grade mixtures of partial glycerides and/or mixtures with glycerides.

5. Composition according to Claims 1 to 4, **characterised in that** component a) and/or component b) each comprise glycerol triesters or partial esters of coconut fatty acids, the mixtures in each case having a melting point in the claimed range.

6. Composition according to Claims 1 to 5, **characterised in that** glycerol partial esters with C12 fatty acids, preferably glycerol monolaurate, is present as component c).

7. Composition according to Claims 1 to 6, **characterised in that** a polyvinyl stearyl ether is present as component c).

8. Composition according to Claims 1 to 7, **characterised in that** it comprises component a) in amounts of from 10 to 60% by weight, component b) in amounts of from 10 to 60% by weight and component c) in amounts of from 10 to 25% by weight.

9. Composition according to Claims 1 to 8, **characterised in that** it has a melting point in the range from 35 to 65°C, preferably from 35 to 50°C and in particular from 35 to 45°C.

10. Composition according to Claims 1 to 9, **characterised in that** it comprises silicone waxes in amounts of from 1 to 6% by weight, preferably 1.5 to 5.5% by weight and in particular from 2 to 5% by weight.

11. Composition according to Claims 1 to 10, **characterised in that** it comprises skin-friendly and/or skincare substances in amounts of from 0.1 to 10% by weight, preferably 1 to 8% by weight and in particular from 2 to 6% by weight.

12. Composition according to Claims 1 to 11, **characterised in that** it comprises water in amounts of from 0.5 to 3% by weight, preferably 0.5 to 2% by weight and in particular 0.5 to 1.5% by weight.

13. Composition according to Claims 1 to 12, **characterised in that** it comprises 50 to 60% by weight of a mixture of glycerol esters of coconut fatty acids having a melting point of from 30 to 33°C as component a), 10 to 20% by weight of a linear, unsaturated fatty alcohol having a melting point of from 57 to 60°C as component b), 15 to 20 parts of polyvinyl stearyl ether having a melting point of from 45 to 48°C as component c), and optionally 2 to 5% by weight of silicone wax, and 5 to 10% by weight of a skincare substance.

14. Use of glycerol monolaurate and/or polyvinyl stearyl ether as crystallization accelerator in lotions for the skin-friendly finishing of nonwovens.

15. Nonwoven comprising polyethylene, **characterised in that** it has been finished using compositions according to Claim 1.

## Patentansprüche

1. Bei 21°C festes Mittel, enthaltend mindestens
a) 5 bis 70 Gew.-% einer im Bereich von 25 bis 37°C schmelzende Komponente, ausgewählt aus der Gruppe der synthetischen Wachse, Paraffine, Fettsäureester, Polyhydroxyfettsäureester, Fettalkohole, alkoxylierten Fettsäureester, alkoxylierten Fettalkohole und Mischungen dieser Verbindungen und
b) 5 bis 70 Gew.-% einer Komponente, deren Schmelzpunkt mindestens 5°C höher ist als der Schmelzpunkt der Komponente a), und die Komponente b) ausgewählt ist aus der Gruppe der Polyhydroxyfettsäureester, C₁₄-C₂₂-Fettalkohole, C₁₂-C₂₂-Fettsäuren, den alkoxylierten Derivaten der Fettalkohole und -ester, sowie Mischungen dieser Komponenten, **dadurch gekennzeichnet, daß** die Mittel zusätzlich als Komponente
c) 5 bis 25 Gew.-% eines Kristallisationsbeschleunigers enthalten, ausgewählt aus der Gruppe der Partialglyceride und polymeren Wachse, mit der Maßgabe, daß der Kristallisationsbeschleuniger einen Schmelzpunkt aufweist, der im Bereich von 45 bis 70°C liegt, und dass die Mittel weniger als 5 Gew.-% Wasser enthalten.

2. Mittel nach Anspruch 1, **dadurch gekennzeichnet, daß** die Komponente b) einen Schmelzpunkt im Bereich von 40 bis 60°C aufweist.

3. Mittel nach den Ansprüchen 1 bis 2, **dadurch gekennzeichnet, daß** die Komponente a) ausgewählt ist aus den Mischungen von Glyceriden von Fettsäuren mit 8 bis 18 C-Atomen, vorzugsweise aus technischen Mischungen von Partialglyceriden und/ oder Abmischungen mit Glyceriden.

4. Mittel nach den Ansprüchen 1 bis 3, **dadurch gekennzeichnet, daß** die Komponente b) ausgewählt ist aus Mischungen von Glyceriden von Fettsäuren mit 8 bis 18 C-Atomen, vorzugsweise aus technischen Mischungen von Partialglyceriden und/oder Abmischungen mit Glyceriden.

5. Mittel nach den Ansprüchen 1 bis 4, **dadurch gekennzeichnet, daß** die Komponente a) und/oder die Komponente b) jeweils Glycerintri- oder -partialester von Kokosfettsäuren enthalten, wobei die Mischungen jeweils einen Schmelzpunkt im beanspruchten Bereich aufweisen.

6. Mittel nach den Ansprüchen 1 bis 5, **dadurch gekennzeichnet, daß** als Komponente c) Glycerinpartialester mit C₁₂-Fettsäuren, vorzugsweise Glycerinmonolaurat enthalten ist.

7. Mittel nach den Ansprüchen 1 bis 6, **dadurch gekennzeichnet, daß** als Komponente c) Polyvinylstearylether enthalten ist.

8. Mittel nach den Ansprüchen 1 bis 7, **dadurch gekennzeichnet, daß** es die Komponente a) in Mengen von 10 bis 60 Gew.-%, die Komponente b) in Mengen von 10 bis 60 Gew.-% und die Komponente c) in Mengen von 10 bis 25 Gew.-% enthält.

9. Mittel nach den Ansprüchen 1 bis 8, **dadurch gekennzeichnet, daß** es einen Schmelzpunkt hat, der im Bereich von 35 bis 65, vorzugsweise von 35 bis 50 und insbesondere von 35 bis 45°C liegt.

10. Mittel nach den Ansprüchen 1 bis 9, **dadurch gekennzeichnet, daß** es Silikonwachse in Mengen von 1 bis 6 Gew.-%, vorzugsweise 1,5 bis 5,5 Gew.-% und insbesondere von 2 bis 5 Gew.-% enthält.

11. Mittel nach den Ansprüchen 1 bis 10, **dadurch gekennzeichnet, daß** es hautfreundliche bzw. hautpflegende Substanzen in Mengen von 0,1 bis 10 Gew.-%, vorzugsweise 1 bis 8 Gew.-% und insbesondere von 2 bis 6 Gew.-% enthält.

12. Mittel nach den Ansprüchen 1 bis 11, **dadurch gekennzeichnet, daß** es Wasser in Mengen von 0,5 bis 3 Gew.-%, vorzugsweise 0,5 bis 2 Gew.-% und insbesondere von 0,5 bis 1,5 Gew.-% enthält.

13. Mittel nach den Ansprüchen 1 bis 12, **dadurch gekennzeichnet, daß** es als Komponente a) 50 bis 60 Gew.-% einer Mischung aus Glycerinestern von Kokosfettsäuren mit einem Schmelzpunkt von 30 bis 33°C enthält, als Komponente b) 10 bis 20 Gew.-% eines linearen, ungesättigten Fettalkohols mit einem Schmelzpunkt von 57 bis 60°C, als Komponente c) 15 bis 20 Teile Polyvinylstearylether mit einem Schmelzpunkt von 45 bis 48°C und optional 2 bis 5 Gew.-% Silikonwachs sowie 5 bis 10 Gew.-% einer hautpflegenden Substanz.

14. Verwendung von Glycerinmonolaurat und/oder Polyvinylstearylether als Kristallisationsbeschleuniger in Lotionen zur hautfreundlichen Ausrüstung von Vliesstoffen.

15. Vliesstoff, enthaltend Polyethylen, **dadurch gekennzeichnet, daß** er mit Mitteln gemäß Anspruch 1 ausgerüstet ist.

## Revendications

1. Composition solide à 21 °C comprenant au moins :
a) 5 à 70 % en poids d'un composant fondant dans la plage allant de 25 à 37 °C, choisi dans le groupe des cires de synthèse, des paraffines, des esters d'acides gras, des esters d'acides gras polyhydroxylés, des alcools gras, des esters d'acides gras alcoxylés, des alcools gras alcoxylés et des mélanges de ces composés, et
b) 5 à 70 % en poids d'un composant dont le point de fusion est d'au moins 5°C supérieur au point de fusion du composant a), le composant b) étant choisi dans le groupe des esters d'acides gras polyhydroxylés, des alcools gras en C₁₄-C₂₂, des acides gras en C₁₂-C₂₂, des dérivés alcoxylés des alcools gras et des esters gras et des mélanges de ces composants,
**caractérisée en ce que**
les compositions comprennent de plus comme composant :
c) 5 à 25 % en poids d'un accélérateur de cristallisation choisi dans le groupe des glycérides partiels et des cires polymères, étant précisé que l'accélérateur de cristallisation a un point de fusion allant de 45 à 70 °C et que les compositions comprennent moins de 5 % en poids d'eau.

2. Composition selon la revendication 1,
**caractérisée en ce que**
le composant b) a un point de fusion dans la plage allant de 40 à 60 °C.

3. Composition selon les revendications 1 et 2,
**caractérisée en ce que**
le composant a) est choisi parmi les mélanges de glycérides d'acides gras ayant 8 à 18 atomes de carbone, de préférence à partir de mélanges de qualité technique de glycérides partiels et/ou de mélanges avec des glycérides.

4. Composition selon les revendications 1 à 3,
**caractérisée en ce que**
le composant b) est choisi parmi des mélanges de glycérides d'acides gras ayant 8 à 18 atomes de carbone, de préférence des mélanges de qualité technique de glycérides partiels et/ou de mélanges avec des glycérides.

5. Composition selon les revendications 1 à 4,
**caractérisée en ce que**
le composant a) et/ou le composant b) comprennent chacun des triesters de glycérol ou des esters partiels d'acides gras de coprah, les mélanges ayant dans chaque cas un point de fusion dans la plage revendiquée.

6. Composition selon les revendications 1 à 5,
**caractérisée en ce que**
le composant c) comporte des esters partiels de glycérol avec des acides gras en C₁₂, de préférence du monolaurate de glycérol.

7. Composition selon les revendications 1 à 6,
**caractérisée en ce que**
le composant c) comporte un polyvinyl stéaryl éther.

8. Composition selon les revendications 1 à 7,
**caractérisée en ce qu'**
elle comprend le composant a) dans des quantités allant de 10 à 60 % en poids, le composant b) dans des quantités allant de 10 à 60 % en poids et le composant c) dans des quantités allant de 10 à 25 % en poids.

9. Composition selon les revendications 1 à 8,
**caractérisée en ce qu'**
elle a un point de fusion dans la plage allant de 35 à 65 °C, de préférence de 35 à 50 °C et en particulier de 35 à 45 °C.

10. Composition selon les revendications 1 à 9,
**caractérisée en ce qu'**
elle comprend des cires de silicone dans des quantités allant de 1 à 6 % en poids, de préférence de 1,5 à 5,5 % en poids et en particulier de 2 à 5 % en poids.

11. Composition selon les revendications 1 à 10,
**caractérisée en ce qu'**
elle comprend des substances agréables pour la peau et/ou de soin pour la peau dans des quantités allant de 0,1 à 10 % en poids, de préférence de 1 à 8 % en poids et en particulier de 2 à 6 % en poids.

12. Composition selon les revendications 1 à 11,
**caractérisée en ce qu'**
elle comprend de l'eau dans des quantités allant de 0,5 à 3 % en poids, de préférence de 0,5 à 2 % en poids et en particulier de 0,5 à 1,5 % en poids.

13. Composition selon les revendications 1 à 12,
**caractérisée en ce qu'**
elle comprend de 50 à 60 % en poids d'un mélange d'esters glycéroliques d'acides gras de coprah ayant un point de fusion allant de 30 à 33°C en tant que composant a), 10 à 20 % en poids d'un alcool gras linéaire insaturé ayant un point de fusion allant de 57 à 60°C en tant que composant b), 15 à 20 parties de polyvinyl stéaryl éther ayant un point de fusion allant de 45 à 48 °C en tant que composant c), et éventuellement 2 à 5 % en poids de cire de silicone et 5 à 10 % en poids d'une substance de soin pour la peau.

14. Utilisation de monolaurate de glycérol et/ou de polyvinyl stéaryl éther en tant qu'accélérateur de cristallisation dans des lotions pour apprêter des non tissés de façon à les rendre agréables pour la peau.

15. Non-tissé comprenant du polyéthylène,
**caractérisé en ce qu'**
il a été apprêté en utilisant des compositions selon la revendication 1.
